# EUROPÄISCHE PATENTSCHRIFT

(11) **EP 3 179 957 B1**
(45) Veröffentlichungstag und Bekanntmachung des Hinweises auf die Patenterteilung: **28.10.2020**
(21) Anmeldenummer: 15759391.4
(22) Anmeldetag: 13.08.2015
(51) Int. Cl.: A61C 8/02, A61C 19/06, A61C 17/02, A61C 8/00

(54) **SYSTEM ZUR IN-SITU-REINIGUNG VON DENTALIMPLANTATEN**
SYSTEM FOR IN SITU CLEANING OF DENTAL IMPLANTS
SYSTÈME POUR LE NETTOYAGE IN SITU D'IMPLANTS DENTAIRES

(30) Priorität: 15.08.2014 DE 102014216294
(43) Veröffentlichungstag der Anmeldung: 21.06.2017
(73) Patentinhaber: Zyfoma GmbH, 64331 Weiterstadt (DE)
(72) Erfinder: BRODBECK, Urs, 8703 Erlenbach (CH)
(74) Vertreter: Tergau & Walkenhorst Patentanwälte PartGmbB
(86) Internationale Anmeldenummer: PCT/EP2015/068705
(87) Internationale Veröffentlichungsnummer: WO 2016/023998

(56) Entgegenhaltungen:
- WO-A1-2007/051339
- WO-A1-2007/051339
- WO-A1-2014/075755
- CH-A5- 561 548
- DE-B3-102012 022 593
- DE-B3-102012 022 593
- KR-A- 20100 056 051

## Beschreibung

Die Erfindung bezieht sich auf ein Reinigungssystem für ein in den Kieferknochen eines Patienten eingebrachtes Dental-Implantat-Teil.

Zum Ausgleich des Verlusts eines Zahns können im Rahmen der rekonstruktiven Therapie Dentalimplantate zum Einsatz kommen. Sie werden üblicherweise an Stelle eines extrahierten oder ausgefallenen Zahnes in den Kieferknochen eingesetzt, um dort nach einer Einheilphase von etwa vier bis zwölf Wochen ein als Zahnersatz dienendes prothetisches Teil oder eine Krone zu halten. Dazu ist ein derartiges Dentalimplantat üblicherweise als geeignet geformter Metallkörper ausgebildet, der durch Einschrauben an der vorgesehenen Stelle in den Kieferknochen eingesetzt wird. Das Dentalimplantat weist dabei in der Regel am apikalen Ende ein zumeist selbstschneidendes Schraubengewinde auf, mit welchem das Dentalimplantat in das entsprechend präparierte Implantatbett eingesetzt wird.

Derartige Dentalimplantate können in einteiliger oder auch mehrteiliger Bauweise ausgeführt sein. Bei der mehrteiligen Bauweise umfasst ein derartiges Dentalimplantat üblicherweise ein erstes, auch als Pfostenteil oder eigentliches Implantat bezeichnetes Dental-Implantat-Teil, das durch Einschrauben an der vorgesehenen Stelle in den Kieferknochen des Patienten eingesetzt wird und dementsprechend das geeignete, meistens selbstschneidende Schraubengewinde in seinem Außenbereich aufweist. Dieses erste Dental-Implantat-Teil ist sodann üblicherweise mit geeigneten Mitteln zum Herstellen einer Verbindung zu einem später hinzuzufügenden, zweiten Dental-Implantat-Teil ausgerüstet, das zum Tragen der eigentlichen prothetischen Versorgung für den Patienten vorgesehen ist. Zur Herstellung der genannten Verbindung zwischen diesen Implantat-Teilen wird üblicherweise auf eine Schraubverbindung zurückgegriffen, wobei das erste, in den Kieferknochen inserierte Dental-Implantat-Teil in seinem Innenbereich als Verbindungsmittel ein Innengewinde aufweist, in das eine Verbindungsschraube eingeschraubt werden kann. An einer derartigen Verbindungsschraube wird sodann das zweite Implantat-Teil am ersten Dental-Implantat-Teil befestigt.

Eine derartige, mehrteilige Ausführung des Implantats hat üblicherweise den Vorteil, dass die Versorgung des Patienten auf mehrere Behandlungs- und Bearbeitungsschritte aufgeteilt werden kann, so dass individuelle Behandlungsabschnitte vergleichsweise kurz und damit für den Patienten schonend gehalten werden können. In einem ersten Behandlungsschritt wird dabei üblicherweise das erste Dental-Implantat-Teil in den Kieferknochen eingesetzt, also eingeschraubt, und kann nach der Insertion über einen gewissen Zeitraum, beispielsweise sechs Wochen, einheilen. Zwischenzeitlich kann das zweite Dental-Implantat-Teil, üblicherweise auch als Abutment bezeichnet, für die spätere Einbringung vorbereitet werden, wobei unter Berücksichtigung beispielsweise der geometrischen Parameter und dergleichen die prothetische Versorgung geeignet angepasst, vorbereitet oder hergestellt werden kann. Nach Ablauf der ersten Einheilphase kann sodann in einem zweiten Behandlungsschritt das Abutment oder zweite Dental-Implantat-Teil über die vorgesehenen Verbindungsmittel, also in der Regel über eine Verbindungsschraube, am Pfostenteil oder ersten Dental-Implantat-Teil befestigt werden, wobei anschließend die prothetische Versorgung passgenau auf das Abutment aufgesetzt werden kann.

Angesichts der mittlerweile vergleichsweise hohen Anzahl der inserierten Implantate im menschlichen Körper sowie deren verhältnismäßig lange Nutzungsdauer wurde ein stetiger Anstieg im Auftreten von biofilmassoziierten Entzündungszuständen des periimplantären Gewebes festgestellt. An der von Gewebe und Gewebeflüssigkeit umschlossenen festen Oberfläche des Implantats bildet sich nämlich ein Biofilm, der von Bakterien besiedelt wird, die letztlich zu chronischen und wiederkehrenden Infektionen führen können. Dieses Erkrankungsbild wird als Periimplantitis bezeichnet. Insbesondere im dentalen Bereich ist ähnlich wie bei der Parodontitis eine Kombination aus vernachlässigter Mundhygiene, Anhaftung von Biofilm an der üblicherweise mikrorauen Oberfläche des Pfostenteils und weiterer Faktoren ursächlich für das Vollbild der Periimplantitis, welches sich durch eine zunehmende Belastung und Zerstörung des Hart- und/oder Weichgewebes auszeichnet. Die Bereiche, an denen sich das Hart- und/oder Weichgewebe zurückzieht, werden dabei in der Regel mit einem Biofilm überzogen.

Sowohl behandelt als auch unbehandelt kann ein Voranschreiten der periimplantären Entzündung zum Verlust des Implantates und zur Beeinträchtigung des Körper- oder Knochengewebes im Bereich der Einbringungsstelle führen. Es ist daher wünschenswert, möglichst frühzeitig nach Feststellung einer derartigen Entzündung geeignete Gegenmaßnahmen einzuleiten. Diese können von einer Optimierung der Mundhygiene bis hin zu therapeutischen Eingriffen in Form chirurgischer Maßnahmen, also insbesondere einer Entnahme des befallenen Implantats und Neueinsetzen eines Austauschimplantats, reichen. Insbesondere letztgenannte Maßnahme ist jedoch sehr belastend für das Gewebe insgesamt und geht oftmals mit massivem Gewebeabbau in der Umgebung der Insertionsstelle einher. Alternative wirksame Maßnahmen zur Bekämpfung existierender oder sich anbahnender Periimplantitis sind daher äußerst wünschenswert.

Aus der DE 10 2012 022 593 und WO 2014/075755 ist ein Konzept zur Bekämpfung oder Eindämmung einer sich anbahnenden Periimplantitis bekannt, mit dem auf besonders wirksame Weise die Bekämpfung des Bakterienbefalls und der Entzündungsherde, die für die Periimplantitis ursächlich sein können, erreichbar ist. Wie sich bei diesem Konzept überraschenderweise herausgestellt hat, ist eine Beaufschlagung des von Bakterien befallenen Implantats mit Strom- oder Spannungspulsen in Kombination mit einer flüssigkeitsbasierten Behandlung des Implantats zur Aufbereitung des Implantats und Abtötung und Entfernung des Bakterienbefalls besonders hochgradig wirksam. Damit ist insbesondere die Entfernung organischer Reste, die nach Abtötung der Bakterien noch an dem Material anhaften und eine Neuansiedlung von Bakterien in unerwünschter Weise begünstigen könnten, besonders wirkungsvoll und zuverlässig ermöglicht.

Der Erfindung liegt nunmehr die Aufgabe zu Grunde, ein Reinigungssystem für ein in den Kieferknochen eines Patienten eingebrachtes Dental-Implantat-Teil anzugeben, mit dem die zuverlässige Nutzung eines kombinierten, sowohl elektrisch- als auch flüssigkeitsbasierten Reinigungskonzepts noch weiter vereinfacht und damit besonders gut einsetzbar gemacht wird.

Diese Aufgabe wird erfindungsgemäß gelöst mit einem Befestigungselement gemäß Anspruch 1.

Die Erfindung geht dabei von der Überlegung aus, dass ein besonders zuverlässiger und auch breitbandig anwendbarer Einsatz des genannten kombinierten Reinigungskonzepts ermöglicht ist, indem die Handhabung des dazu vorgesehenen Reinigungssystems, insbesondere dessen Montage und Demontage, besonders einfach gehalten wird. Insbesondere sollte das Reinigungssystem derart einfach montierbar und demontierbar ausgeführt sein, dass auch ein Einsatz für ein im Patientenmund inseriertes Implantatteil möglich ist, ohne den Patienten bei der Behandlung einer unzumutbaren Belastung auszusetzen. Dabei wird vorliegend in besonderem Maße angestrebt, die Endmontage besonders einfach zu halten, so dass die entsprechenden Reinigungszeiten im Patientenmund vergleichsweise kurz gehalten werden können.

Um dies zu ermöglichen, ist vorgesehen, das Reinigungssystem mehrteilig auszuführen. Einerseits umfasst das Reinigungssystem dabei ein Befestigungselement, das mit geeigneten Mitteln zum Befestigen am im Patientenmund inserierten Dental-Implantat-Teil oder Pfostenteil ausgerüstet ist. Als Befestigungs- oder Verbindungsmittel in diesem Sinne könnte beispielsweise ein Bajonettverschluss oder dergleichen vorgesehen sein. Unter Rückgriff auf die für mehrteilige Dentalimplantate üblicherweise verwendeten Schraubverbindungen ist das Befestigungselement dabei besonders bevorzugt endseitig mit einem geeignet dimensionierten und an das Innengewinde am Pfostenteil angepassten Außengewinde versehen, so dass das Befestigungselement in das Gewinde im inserierten Dental-Implantat-Teil einschraubbar ist. Die Montage des Befestigungselements am inserierten Pfostenteil kann somit durch einfaches Einschrauben erfolgen, was im Hinblick auf die ansonsten vergleichsweise einfach gehaltene Bauform des Befestigungselements ein vergleichsweise schnell zu erledigender und einfach zu handhabender und damit mit nur geringen Belastungen für den Patienten verbundener Vorgang ist.

Als weitere Komponenten umfasst das Reinigungssystem zudem noch jeweils eine Komponente für die Zuführung der Reinigungsflüssigkeit und eine Komponente zur Herstellung eines elektrischen Strompfads, über den das behandlungsbedürftige, inserierte Dental-Implantat-Teil mit einer externen Stromquelle verbindbar ist. Um auch für diese Komponenten und damit für das Reinigungssystem insgesamt die Endmontage besonders einfach zu gestalten, sind diese beiden Komponenten auf das Befestigungselement aufsteckbar. Die beiden Komponenten können dabei als separate Bauteile ausgeführt sein oder aber auch in der Art einer integrierten Bauweise jeweils Teil eines einzigen, als solches auf das Befestigungselement aufsteckbaren Bauteils sein.

In seiner Gesamtheit erlaubt ein derartig ausgestaltetes Reinigungssystem somit eine besonders einfach gehaltene und damit mit kurzen Bearbeitungszeiten einhergehende Endmontage, da der Einschraubvorgang, der notwendigerweise mit einer Drehbewegung verbunden ist, auf ein besonders einfach gehaltenes und damit leicht bedienbares Bauteil, nämlich das Befestigungselement, beschränkt bleibt. Die komplizierter gestalteten Komponenten, nämlich das elektrische Kontaktelement und das Verteilerelement für die Reinigungsflüssigkeit, die jeweils mit Zuleitungen, Kanälen, Leitungen oder dergleichen verbunden sind und damit Drehbewegungen behindern würden, sind hingegen in axialer Richtung auf das Befestigungselement aufsteckbar und damit ohne größere Behinderungen durch Zuleitungen und dergleichen montierbar.

Konzeptbedingt ist das Reinigungssystem vorteilhafterweise dafür ausgelegt, im montierten Zustand das Dental-Implantat-Teil einer kombinierten Behandlung sowohl auf Basis einer elektrischen Bestromung als auch auf Basis einer Reinigung mit einer Reinigungs- oder Spülflüssigkeit zu unterziehen. Dabei ist vorzugsweise vorgesehen, das Dental-Implantat-Teil als Elektrode in einem elektrischen Strompfad auszubilden und dementsprechend mit einer externen Stromquelle zu kontaktieren, so dass der zu Reinigungszwecken vorgesehene Stromfluss durch die Oberfläche des Dental-Implantat-Teils hindurch erfolgen kann. Um dies auf besonders einfache Weise zu ermöglichen, weist das Befestigungselement in vorteilhafter Ausgestaltung einen elektrisch leitfähigen, vorzugsweise metallischen, Grundkörper auf, der mit einer elektrisch isolierenden Oberflächenbeschichtung versehen ist. Im Bereich des Außengewindes, mit dem das Befestigungselement in das Dental-Implantat-Teil einschraubbar ist, soll hingegen vorzugsweise eine unbeschichtete, elektrisch leitfähige Außenoberfläche vorliegen, so dass über das eingreifende Gewinde die Herstellung eines elektrischen Kontakts zwischen dem Befestigungselement und dem Dental-Implantat-Teil möglich ist. An seinem dem Dental-Implantat-Teil abgewandten Ende kann das Befestigungselement dann in geeigneter Weise eine freiliegende, elektrisch leitfähige und von außen kontaktierbare Teiloberfläche aufweisen, über die der elektrische Kontakt mit dem Kontaktelement und über dieses mit der externen Stromquelle hergestellt werden kann. Auf diese Weise ist die elektrische Verbindung des Dental-Implantat-Teils mit der externen Stromquelle über das Befestigungselement und das elektrische Kontaktelement möglich.

Das Verteilerelement ist für die Zuführung und Verteilung der Reinigungsflüssigkeit im Raumbereich in unmittelbarer Umgebung des Dental-Implantat-Teils vorgesehen. Zu diesem Zweck ist das Verteilerelement vorzugsweise ringförmig ausgeführt und weist einen medienseitig mit dem Medienkanal verbindbaren Medienringkanal auf, der mit einer Anzahl von, also einer oder mehreren, vorzugsweise über den Umfang verteilten Medienaustrittsöffnungen versehen ist. Durch die ringförmige Ausgestaltung des Verteilerelements kann dieses beim Aufstecken auf das Befestigungselement in der Art einer konzentrischen Positionierung derart auf das Dental-Implantat-Teil aufgesetzt werden, dass die Medienaustrittsöffnungen um den Außenumfang des Dental-Implantat-Teils herum positioniert sind, so dass die Beaufschlagung des gesamten Außenumfangs des Dental-Implantat-Teils mit Reinigungsflüssigkeit ermöglich ist. Um dabei eine besonders gezielte Zuführung der Reinigungsflüssigkeit zu ermöglichen, ist das Verteilerelement in besonders vorteilhafter Ausgestaltung derart dimensioniert und ausgeführt, dass sich die Medienaustrittsöffnungen in einem Abstand von nicht mehr als 10 mm, vorzugsweise nicht mehr als 5 mm, besonders bevorzugt nicht mehr als 3 mm von der äußeren Oberfläche des Dental-Implantat-Teils befinden.

Im Hinblick auf die vorgesehene Ausgestaltung der gezielten elektrischen Stromführung bei am Dental-Implantat-Teil montierten Reinigungssystem weist das Verteilerelement in zweckmäßiger Ausgestaltung ein elektrisch isolierendes Außengehäuse auf. Dieses ist bevorzugt aus einem Kunststoff gefertigt.

Um bei der Behandlung des Dental-Implantat-Teils eine besonders zuverlässige und effiziente Wirkungsweise zu erreichen, ist in besonders vorteilhafter Ausgestaltung vorgesehen, bei der Bestromung des Dental-Implantat-Teils den Strom oder Strompuls durch die zugeführte Reinigungsflüssigkeit und/oder durch den für diese vorgesehenen Medienkanal zu leiten. Dabei liegt die Überlegung zu Grunde, dass als Reinigungsflüssigkeit in ganz besonders bevorzugter Ausgestaltung die Reinigungsflüssigkeit vorgesehen ist, wie sie in ihren Bestandteilen und Varianten aus der DE 10 2012 022 593 bekannt ist. Deren Offenbarungsgehalt wird bzgl. Art und Zusammensetzung der Reinigungsflüssigkeit sowie deren wesentlicher Bestandteile und Zusammensetzungsvarianten vollumfänglich miteinbezogen.

Auf Grund ihres Salz- bzw. lonenanteils ist eine derartige Reinigungsflüssigkeit elektrisch leitfähig, so dass die Flüssigkeitssäule innerhalb des Medienkanals in der Art einer Zusatznutzung auch als stromführendes Element verwendet werden kann. Bei dieser besonders bevorzugten Zusatznutzung der Flüssigkeitssäule erfolgt der Stromfluss einerseits über die Stromleitung, das elektrische Kontaktelement und das Befestigungselement zum Dental-Implantat-Teil, welches somit bei der Bestromung als Elektrode dient. Andererseits erfolgt der Stromfluss, ausgehend von der Oberfläche des inserierten Dental-Implantat-Teils, über die dieses umgebende, die Oberfläche benetzende Reinigungsflüssigkeit, das Verteilerelement und den Medienkanal über die in diesem befindliche Flüssigkeitssäule. Bedarfsweise kann, gegebenenfalls zur Verbesserung der elektrischen Leitungseigenschaften, in vorteilhafter Ausgestaltung im Medienkanal ein stromleitendes Kabel integriert sein.

Auf Grund der für die Montage des Reinigungssystems, also für die Anbringung von Verteilerelement einerseits und elektrischem Kontaktelement andererseits am oder auf dem Befestigungselement, vorgesehene Steckverbindung - in der Art eines Aufsteckens - ist das Reinigungssystem für eine besonders einfach gehaltene Endmontage im Patientenmund besonders geeignet. Um vor diesem Hintergrund die Einfachheit und Betriebssicherheit des Systems noch weiter zu verbessern und insbesondere auch höchsten hygienischen und Versorgungsansprüchen bei der Patientenbehandlung zu genügen, sind in ganz besonders bevorzugter Ausgestaltung das Verteilerelement und/oder das elektrische Kontaktelement, als Einwegprodukt(e) ausgebildet. Gerade durch eine derartige Ausführung als Einwegprodukte, die als eigenständig erfinderisch angesehen wird, kann nämlich sichergestellt werden, dass ohne weitere Sicherungsmaßnahmen höchste hygienische Ansprüche erfüllt werden, wobei gleichzeitig die Handhabung des Gesamtsystems besonders einfach gehalten werden kann.

Die Auslegung als Einwegprodukt spezifiziert dabei insbesondere, dass das jeweilige Element hinsichtlich der Wahl der eingesetzten Materialien und/oder der eingesetzten Herstellungs- oder Bearbeitungsverfahren besonders kostengünstig ausgeführt und lediglich zum einmaligen Gebrauch vorgesehen ist. Insbesondere kann ein als derartiges Einwegprodukt ausgestaltetes Element in besonders bevorzugter Weiterbildung für seine irreversible Zerstörung bei der Entnahme oder Demontage des Reinigungssystems ausgelegt sein. Damit ist sichergestellt, dass eine unerwünschte Mehrfachverwendung, die zwingend eine aufwändige Reinigungs- und Desinfektionsprozedur erfordern würde, systembedingt ausgeschlossen bleibt. Eine derartige Auslegung kann insbesondere bedingen, dass das jeweilige Element nicht zerstörungsfrei demontiert werden kann. Dies kann beispielsweise dadurch erreicht werden, dass an geeigneten Stellen des jeweiligen Elements Versiegelungen oder Umhüllungen oder dergleichen vorgesehen sind, die bei der Montage oder bei der Demontage aufgebrochen, zerrissen oder auf sonstige Weise beschädigt werden, so dass für den Verwender bereits im Vorfeld der Montage unmittelbar erkennbar ist, ob es sich bei dem jeweiligen Element um ein noch unbenutztes handelt.

Der in das Verteilerelement mündende Medienkanal für die Reinigungsflüssigkeit ist an seiner Innenoberfläche mit einer elektrisch leitenden metallischen Beschichtung versehen. Diese kann einerseits zu den elektrischen Leitungseigenschaften des Medienkanals und somit zu dessen ordnungsgemäßer Funktion als elektrische Stromzuleitung beitragen.

Die metallische Beschichtung ist als metallische Opferschicht vorgesehen, die bei der Benutzung des jeweiligen Verteilerelements von der Reinigungsflüssigkeit sukzessive aufgelöst wird, so dass die jeweiligen Ionen in die Reinigungsflüssigkeit eingebracht werden. Durch diese Ausführung einer metallischen Beschichtung als Opferschicht, die als erfinderisch angesehen wird, ist einerseits mit besonders einfachen Mitteln eine Einspeisung von Metallionen in die Reinigungsflüssigkeit während deren Transport durch den Medienkanal ermöglicht, so dass die Anreicherung der Reinigungsflüssigkeit mit den am Behandlungsort, also an der Oberfläche des inserierten Dental-Implantat-Teils, für die Reinigung erforderlichen oder vorgesehenen Ionen sichergestellt werden kann. Andererseits gewährleistet die Ausführung der Beschichtung als metallische Opferschicht, die sich nach einer gewissen Betriebsdauer auflöst, aber auch eigenständig und auf besonders einfache Weise die besonders bevorzugt vorgesehene Auslegung der jeweiligen Komponenten als Einwegprodukt, da nach dem "Verbrauch" der Opferschicht die Wirksamkeit des Reinigungssystems nicht mehr ohne Weiteres gegeben ist.

Bei der Ausgestaltung dieser Metallschicht als Opferschicht, also als vergleichsweise dünn gehaltene Beschichtung, die nach einer gewissen Betriebsdauer auf Grund der fortschreitenden loneneinlagerung in die Reinigungsflüssigkeit verschwindet, ist somit auf besonders einfache Weise die Auslegung des Verteilerelements als Einwegprodukt ermöglicht, da die ordnungsgemäße und konzeptgemäße Funktionsweise des Verteilerelements vom loneneintrag aus der Opferschicht in die Reinigungsflüssigkeit abhängig ist. Besonders bevorzugt ist eine derartige Opferschicht als Beschichtung auf Reintitan als Basismaterial für den jeweiligen Medienkanal vorgesehen.

Die Beschichtung, die in der genannten Art als Opferschicht ausgeführt ist, ist dabei besonders bevorzugt nach den Kriterien ausgelegt, dass das Beschichtungsmaterial bei Beschickung mit der Reinigungsflüssigkeit in Lösung geht, um in der Reinigungsflüssigkeit den gewünschten lonenanteil bereitzustellen und den angestrebten Stromfluss zu ermöglichen. Des Weiteren sollten die Materialien der Opferschicht körperverträglich sein, d. h. im menschlichen Körper in den freigesetzten Mengen keine schädlichen Wirkungen entfalten, und sie sollten sich nicht metallisch auf dem Implantatkörper abscheiden. Besonders bevorzugt umfasst die Opferschicht als Bestandteile daher Mangan, Zink und/oder Kupfer und/oder in ganz besonders bevorzugter Ausführung Magnesium und/oder Aluminium.

Durch eine derartige Ausgestaltung wird eine besonders bevorzugte und als eigenständig erfinderisch angesehene Verwendung des Verteilerelements in Kombination mit einem Elektrolyten, umfassend Zitronensäure, Milchsäure, Äpfelsäure und/oder Essigsäure, ermöglicht. Durch diese besonders bevorzugte kombinierte Verwendung entsteht durch den loneneintrag aus der Opferschicht in den Elektrolyten hinein die eigentlich im inserierten Bereich des Implantats wirksame Reinigungs- und Behandlungsflüssigkeit, die zusätzlich zu den genannten Säureanteilen den lonenanteil aus Magnesium, Aluminium, Kalium und/oder Calcium umfasst, und die in ihren Bestandteilen und Varianten aus der DE 10 2012 022 593 bekannt ist. Gerade eine diese Bestandteile umfassende Reinigungsflüssigkeit hat sich in überraschender Weise als besonders zuverlässig und wirksam bei der Behandlung des inserierten Implantat-Teils erwiesen. Vorzugsweise kann dabei der dem Verteilerelement zugeführte Elektrolyt auch bereits von vornherein Zusatzanteile eines Metallsalzes, umfassend Magnesium-, Aluminium-, Kalium- und/ oder Calciumionen, enthalten.

In einem ebenfalls als eigenständig erfinderisch angesehenen Verfahren zum Reinigen eines Implantat-Teils von anhaftendem Biofilm wird ein Elektrolyt, umfassend Zitronensäure, Milchsäure, Äpfelsäure und/oder Essigsäure, über einen mit einer metallischen Opferschicht versehenen Medienkanal geführt und dabei mit Metallionen, besonders bevorzugt mit Magnesium- oder Aluminium-Ionen, angereichert, bevor der solchermaßen mit Metallionen angereicherte Elektrolyt unter Verwendung des zu reinigenden Implantat-Teils als Elektrode mit einem Stromfluss beaufschlagt wird.

Hinsichtlich der mechanischen Eigenschaften bei der Ausgestaltung der Steckverbindung für Kontaktelement und Verteilerelement auf das Befestigungselement ist vorzugsweise eine Art von Rast- oder Clipsverbindung vorgesehen. Insbesondere weist das Außengehäuse des Verteilerelements vorteilhafterweise einen auf eine korrespondierende umlaufende Wulst des Befestigungselements aufsteckbaren Rastkragen auf.

Das elektrische Kontaktelement weist hingegen in besonders vorteilhafter Ausgestaltung einen elektrisch leitend mit der Stromleitung verbundenen, mit einer Anzahl von Kontaktierungsspitzen versehenen Kontaktklemmring auf. Dieser ist vorzugsweise derart ausgelegt und dimensioniert, dass sich die Kontaktspitzen beim Aufsetzen oder Aufstecken auf das Befestigungselement verbiegen und damit für einen besonders zuverlässigen Halt des aufgesteckten Elements am Befestigungselement sorgen. Damit ist einerseits eine zuverlässige elektrische Kontaktierung zwischen Kontaktelement und Befestigungselement möglich, wobei anderseits auf Grund dieser Verbiegung in besonders einfacher Weise die Ausgestaltung als Einwegprodukt für das Kontaktelement ermöglicht ist. Insbesondere ist nach einmaliger Benutzung des Kontaktelements auf Grund der entstandenen Verbiegung der Rastzähne oder Kontaktierungsspitzen für den Verwender eindeutig und unzweifelhaft erkennbar, dass das jeweilige Element bereits einmal benutzt wurde und daher für einen erneuten Einsatz nicht geeignet ist.

Besonders bevorzugt sind einige oder alle der zur Stromführung vorgesehenen Bestandteile des Reinigungssystem aus Titan (Ti), bevorzugt Reintitan (grade 1 bis 4), aus Zirkonium (Zr), aus Tantal (TI) oder einer Legierung mit einigen oder allen diesen Bestandteilen als Basismaterial gefertigt, da dieses aufgrund seiner hohen Biokompatibilität besonders zuverlässig für die genannten Zwecke einsetzbar ist. Um dabei auch im Hinblick auf die bevorzugt vorgesehene Ausgestaltung als Einwegprodukt auf einfache Weise eine besonders hohe Bediensicherheit zu gewährleisten, sind diese Komponenten besonders bevorzugt in der Art einer gekapselten Ausführung mit einer wasserlöslichen Außenbeschichtung, beispielsweise aus Zucker, versehen, die sich im Kontakt mit der Reinigungsflüssigkeit oder einer Körperflüssigkeit auflöst und das eigentliche Elektrodenmaterial freigibt. Die Außenbeschichtung kann dabei direkt auf dem Basismaterial oder gegebenenfalls auch auf der auf diesem vorgesehenen Opferschicht, in der Art eines Schichtpakets, aufgebracht sein.

Die mit der Erfindung erzielten Vorteile bestehen insbesondere darin, dass auf Grund der mehrteiligen Ausführung des Reinigungssystems und der funktionalen Trennung bei der Montage der einzelnen Elemente (Drehbewegung beim Einschrauben des Befestigungselements in das Gewinde im Dental-Implantat-Teil einerseits und axiale Steckbewegung beim Aufbringen der mit Leitungen, Zuführungen und dergleichen versehenen elektrischen Kontakt- und Verteilerelemente) eine in besonderem Maße erleichterte Montage des Systems auch für im Patientenmund verbliebene, inserierte Dental-Implantat-Teile ermöglicht ist. Dies kann mit besonders gering gehaltenen Montagezeiten erfolgen.

Ein Ausführungsbeispiel der Erfindung wird anhand einer Zeichnung näher erläutert.

Darin zeigen:
- FIG. 1: ein mehrteiliges Dentalimplantat im montierten Zustand,
- FIG. 2: das Dentalimplantat gemäß FIG. 1 in Explosionsdarstellung,
- FIG. 3: ein Reinigungssystem für das Pfostenteil des Dentalimplantats gemäß FIGs. 1, 2 im montierten Zustand im Längsschnitt,
- FIG. 4: das Reinigungssystem gemäß FIG. 3 in Explosionsdarstellung im Längsschnitt,
- FIG. 5: ein Kontaktelement des Reinigungssystems gemäß FIGs. 3, 4 im Längsschnitt (FIG. 5A) und in Draufsicht (FIG. 5B),
- FIG. 6: ein Verteilerelement des Reinigungssystems gemäß FIGs. 3, 4 in Unteransicht (FIG. 6A) und im Längsschnitt (FIG. 6B), und
- FIG. 7: einen Verteilereinsatz des Verteilerelements gemäß FIG. 6 in Draufsicht.

Gleiche Teile sind in allen Figuren mit denselben Bezugszeichen versehen.

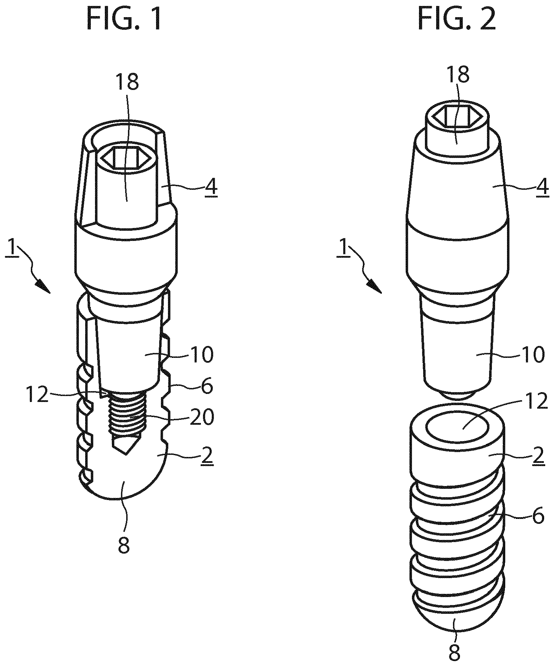

Das Dental-Implantatsystem 1 gemäß FIG. 1 ist zum Einsatz in den Kieferknochen anstelle eines extrahierten oder ausgefallenen Zahnes vorgesehen, um dort ein als Zahnersatz dienendes prothetisches Teil oder eine Krone zu halten. Das Dental-Implantatsystem 1 ist dabei mehrteilig ausgeführt und umfasst ein als so genanntes Pfostenteil ausgeführtes erstes Dental-Implantat-Teil 2 und ein diesem zugeordnetes, zur Anbringung eines Zahnersatzstückes vorgesehenes, auch als Aufbauteil oder Abutment bezeichnetes zweites Dental-Implantat-Teil 4. Das erste Dental-Implantat-Teil 2 oder Pfostenteil ist dabei außenseitig mit einem Außengewinde 6 versehen, das insbesondere am apikalen Ende 8 als selbstschneidendes Schraubengewinde ausgestaltet ist. Damit kann das erste Dental-Implantat-Teil 2 oder Pfostenteil durch Einschrauben an der vorgesehenen Stelle in den Kieferknochen eingesetzt werden.

Um nach geeigneter Anbringung des Zahnersatzstücks oder der Prothese am Aufbauteil oder zweiten Dental-Implantat-Teil 4 ein Einbringen in das Pfostenteil oder erste Dental-Implantat-Teil 2 bei hoher mechanischer Stabilität zu ermöglichen, ist an das zweite Dental-Implantat-Teil 4 ein Verbindungszapfen 10 angeformt, der in einen zugeordneten, im ersten Dental-Implantat-Teil 2 vorgesehenen Aufnahmekanal 12 einschiebbar ist. Durch das Einschieben des Verbindungszapfens 10 in den Aufnahmekanal 12 entsteht eine mechanische Verbindung der Implantat-Teile 2, 4 miteinander. Für eine hohe mechanische Stabilität ist dabei der Verbindungszapfen 10 in seiner Außenkontur an die Innenkontur des Aufnahmekanals 12 angepasst, wobei beide in Längsrichtung gesehen konisch geformt sein können. Zudem kann die Außenkontur des Verbindungszapfens 10 - und dementsprechend angepasst die Innenkontur des Aufnahmekanals 12 - im Querschnitt mit einer mehrzähligen Symmetrie ausgestaltet sein, so dass beim Zusammenfügen der genannten Komponenten ein rotatorisches Gesperre entsteht und damit eine zuverlässige rotatorische Ausrichtung des Aufbauteils relativ zum Pfostenteil eingestellt werden kann.

Das Dental-Implantatsystem 1 ist mit Verbindungsmitteln zur Herstellung der Verbindung der Implantat-Teile 2, 4 miteinander ausgerüstet. Dabei kann es sich grundsätzlich um beliebige, hierfür geeignete Verbindungsmittel wie beispielsweise einen Bajonettverschluss oder dergleichen handeln. Im Hinblick auf derzeitig gängige Bauformen für Dentalimplantate ist im Ausführungsbeispiel als derartiges Verbindungsmittel in der Art einer besonders bevorzugten Ausführungsform eine Schraubverbindung vorgesehen. Dazu ist eine Verbindungsschraube 18 vorgesehen, die in ein innerhalb des ersten Dental-Implantat-Teils 2 vorgesehenes Schraubgewinde 20 eingreift. Hinsichtlich ihrer Materialwahl sind die Implantat-Teile 2, 4 geeignet an den Einsatzzweck angepasst und insbesondere aus einem geeignet gewählten Metall wie beispielsweise Titan gefertigt.

Generell besteht bei Dental-Implantatsystemen, insbesondere auch bei zweiteiligen Implantatsystemen der vorstehend beschriebenen Art, das Problem, dass durch ein Eindringen von Bakterien oder Keimen in den Gewebebereich in der Nähe der Insertionsstelle, insbesondere im Bereich des in den Kiefer eingebrachten Außengewindes 6, Entzündungen oder Entzündungsherde entstehen können. Derartige, insbesondere auch in Folge einer so genannten Periimplantitis entstehende Entzündungen können, insbesondere wenn sie sich über einen längeren Zeitraum entwickeln und verfestigen können, zu einer gravierenden Beeinträchtigung des Gewebes und des Knochens im Bereich der Insertionsstelle führen. Ohne geeignete Gegenmaßnahmen können diese Beeinträchtigungen dazu führen, dass das gesamte Implantatsystem, also insbesondere auch das bereits inserierte Pfostenteil oder erste Dental-Implantat-Teil 2, wieder aus dem Knochen entfernt und durch andere Prothetik ersetzt werden muss. Dieser durch die Periimplantitis hervorgerufene, äußerst unerwünschte Effekt kann somit zu einem Totalverlust des Implantatsystems führen, so dass erneute chirurgische Maßnahmen wie beispielsweise ein Ausschaben des betroffenen Bereichs im Kieferknochen und die Neuversorgung mit einem Implantatsystem notwendig werden können. Durch eine derartige Entnahme kann es zudem zu Knochenverlust oder sonstigem Verlust an Gewebesubstanz kommen, die im Extremfall so weit führen kann, dass eine Neuversorgung mit einem anderen Implantat gar nicht mehr möglich ist. Eine derartige durch Periimplantitis hervorgerufene Notwendigkeit einer Neuversorgung kann auch nach vergleichsweise langen Zeiträumen nach dem ersten Einsetzen des Implantatsystems von beispielsweise bis zu einigen Jahren oder sogar Jahrzehnten auftreten.

Die im Zusammenhang mit einer Periimplantitis beobachteten Keime oder Bakterien können dabei grundsätzlich das Innere des Pfostenteils 2 besiedeln, haften aber in der Regel bevorzugt direkt an der Oberfläche des in den Kieferknochen inserierten Pfostenteils 2 im Kontaktbereich mit dem umgebenden Gewebe oder Knochenmaterial an, also insbesondere im Bereich des Außengewindes 6. In dessen Bereich kann die Oberfläche des Pfostenteils 2 mit einer Aufrauhung oder dergleichen versehen sein, um das Einwachsen in das Gewebe oder den Knochen besonders zu begünstigen und das Einheilen des Pfostenteils 2 nach der Insertion zu unterstützen. Gerade im Bereich einer derartigen, eigentlich als besonders günstig für das Implantatsystem angesehenen Aufrauhung der Oberfläche kann aber die Ansiedlung der Keime oder Bakterien vermehrt stattfinden, wobei die Rauigkeit ein gezieltes Entfernen der vorhandenen Keime oder Bakterien noch zusätzlich erschwert.

Es besteht daher der dringende Wunsch nach geeigneten Gegenmaßnahmen, um für den Fall einer sich anbahnenden oder bereits aufgetretenen Periimplantitis unter Erhaltung des bereits eingesetzten Implantatsystems, also insbesondere des bereits inserierten Pfostenteils 2, wirksam den Entzündungsherd bekämpfen und die eingedrungenen Keime abtöten zu können, so dass sich anschließend wieder gesundes Gewebe oder gesunde Knochensubstanz im Bereich um das Außengewinde 6 herum bilden kann. Dazu ist wünschenswert, zusätzlich zu einem gezielten Abtöten der Keime oder Bakterien im betroffenen Bereich auch noch deren Materialreste und Fragmente zuverlässig aus dem betroffenen Raumbereich zu entfernen, so dass anschließend der betroffene Bereich wieder von gesundem Gewebe oder Knochenmaterial ausgefüllt werden und sich wieder eine innige Verbindung zwischen der Außenoberfläche des Pfostenteils 2 und dem umgebenden Gewebe oder Knochenmaterial bilden kann. Zudem sollte der von der Bakterienbeschichtung gebildete Biofilm einschließlich der organischen Reste abgetöteter Bakterien zuverlässig entfernt werden.

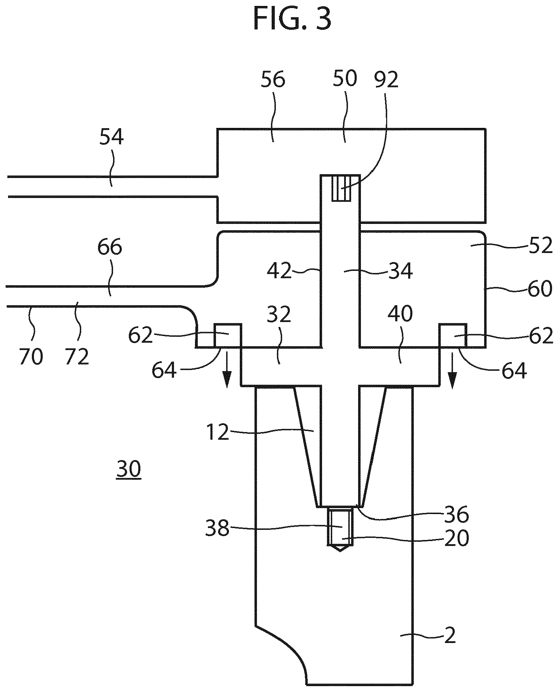

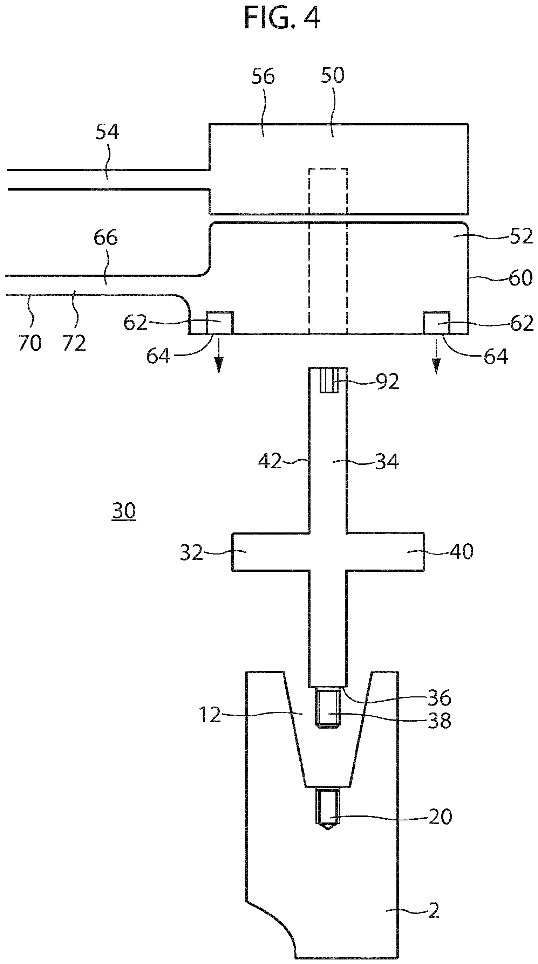

Zu diesem Zweck, also zum Abtöten von Keimen oder Bakterien im Insertionsbereich des Pfostenteils 2 und insbesondere auch zum anschließenden Ausspülen, Entfernen und Austragen der Gewebe- und Materialreste der abgetöteten Bakterien, ist ein Bearbeitungs- oder Reinigungssystem 30 vorgesehen, wie es im Längsschnitt in FIG. 3 und in Explosionsdarstellung in FIG. 4 gezeigt ist. Das Reinigungssystem 30 ist dabei gezielt dafür ausgelegt, das erste Dental-Implantat-Teil 2 oder Pfostenteil nach einer vorherigen Entfernung des zweiten Dental-Implantat-Teils 4 oder Abutments im inserierten Zustand im Patientenmund zu behandeln und von einer Keim- oder Bakterienbesiedlung zu reinigen, indem gezielt auf eine kombinierte Behandlung von elektrischer Bestromung einerseits und den Einsatz einer geeignet gewählten Reinigungsflüssigkeit andererseits zurückgegriffen wird. Das Reinigungssystem 30 ist dabei für eine besonders hohe Bedien- und Montagefreundlichkeit ausgelegt, so dass die eigentliche Bearbeitungszeit im Patientenmund besonders kurz und die Belastung des Patienten besonders gering gehalten werden kann.

Dazu ist, wie dies den Darstellungen in FIG. 3 und FIG. 4 entnehmbar ist, das Reinigungssystem 30 mehrteilig ausgeführt, wobei die einzelnen Bestandteile derart gewählt sind, so dass sich eine besonders hohe Montagefreundlichkeit ergibt. Das Reinigungssystem 30 umfasst dabei zum einen ein Befestigungselement 32, das zur Fixierung der weiteren Komponenten am inserierten Pfostenteil oder ersten Dental-Implantat-Teil 2 vorgesehen und ausgestaltet ist. Das Befestigungselement 32 umfasst dabei einen länglich ausgedehnten, im Ausführungsbeispiel im Wesentlichen zylinderförmigen Grundkörper 34, der an seinem Ende 36 mit einem Außengewinde 38 versehen ist. Das Außengewinde 38 ist dabei zum Einschrauben in das Schraubgewinde 20 im Pfostenteil oder ersten Dental-Implantat-Teil 2 vorgesehen und dementsprechend hinsichtlich seiner Dimensionierung, Gewindeparameter und dergleichen an das Schraubgewinde 20 geeignet angepasst. Der Grundkörper 34 ist ansonsten im Ausführungsbeispiel im Wesentlichen zylindrisch ausgestaltet und in seiner Dimensionierung derart gewählt, dass er problemlos in den eigentlich für den Verbindungszapfen 10 vorgesehenen Aufnahmekanal 12 eingeschoben werden kann. Grundsätzlich könnte der Grundkörper 34 dabei in seinem Endbereich auch konisch ausgeführt und damit geeignet an die Kontur des Aufnahmekanals 12 angepasst sein.

Um das Eindrehen oder Einschrauben des Befestigungselements 32 in das Schraubgewinde 20 zu erleichtern, ist am Grundkörper 34 eine umlaufende Wulst oder Ringscheibe 40 angeordnet, die beim Einschrauben als Handhabe dienen kann. Um die für die eigentliche Behandlung des Pfostenteils oder ersten Dental-Implantat-Teils 2 vorgesehene elektrische Kontaktierung des Pfostenteils zum Zweck der Bestromung zu ermöglichen, ist der Grundkörper 34 des Befestigungselements 32 aus elektrisch leitfähigem, im Ausführungsbeispiel metallischem Material gefertigt und seinen freiliegenden Außenflächen mit einer elektrisch isolierenden Oberflächenbeschichtung 42 versehen.

Ansonsten ist das Befestigungselement 32 im Wesentlichen freigehalten von weiteren Bestandteilen, so dass auf Grund der einfachen Bauform insbesondere ein besonders erleichtertes Eindrehen oder Einschrauben des Befestigungselements 32 in das Schraubgewinde 20 ermöglicht ist, ohne dass sperrige Komponenten dies behindern würden. Das Befestigungselement 32 dient im montierten Zustand zur Befestigung der weiteren, aktiven Komponenten des Reinigungssystems 30, die zur Montage in axialer Richtung des Grundkörpers 34 gesehen auf das Befestigungselement 32 aufgesteckt werden können. Die Montage dieser Komponenten ist somit unter Vermeidung von Dreh- oder Rotationsbewegungen möglich, so dass Strom- oder Medienleitungen, die von den jeweiligen Komponenten abgehen, die Montage oder Demontage nicht oder nur geringfügig behindern.

Als aktive Komponenten für die Durchführung der eigentlichen Reinigung sind ein elektrisches Kontaktelement 50 und ein Verteilerelement 52 für eine Reinigungsflüssigkeit vorgesehen, die beide für eine besonders einfach gehaltene Montage auf das Befestigungselement 32 aufsteckbar sind. Im Ausführungsbeispiel sind das Kontaktelement 50 und das Verteilerelement 52 dabei als separate Bauteile ausgeführt; bedarfsweise und abhängig vom vorgesehenen Anwendungsfall könnte aber auch ein integriertes Bauteil vorgesehen sein, das diese beiden Elemente in einer gemeinsamen Einheit enthält.

Das elektrische Kontaktelement 50 ist mit einer an eine externe Stromquelle anschließbaren Stromleitung 54 verbunden, die ihrerseits über geeignet im Kontaktkopf 56 angeordnete, nicht näher dargestellte Kontaktzungen bei auf das Befestigungselement 32 aufgestecktem Kontaktelement 50 mit dem metallischen, elektrisch leitenden Grundkörper 34 des Befestigungselements 32 verbindbar ist. Auf diese Weise ist bei in das Pfostenteil 2 eingeschraubtem Befestigungselement 32 und auf dieses aufgestecktem Kontaktelement 50 eine elektrisch leitende Verbindung von der externen Stromquelle zum Pfostenteil oder ersten Dental-Implantat-Teil 2 über die Stromleitung 54, das Kontaktelement 50 und den Grundkörper 34 des Befestigungselements 32 herstellbar.

Das Verteilerelement 52 ist dafür ausgelegt, eine von einem externen Medienreservoir zugeführte Reinigungsflüssigkeit in räumlicher Nachbarschaft des zu behandelnden Pfostenteils 2 auszubringen und dessen Oberfläche zu spülen. Des Weiteren soll die vorgesehene Bestromung bei der Behandlung des Pfostenteils 2 unter Nutzung der Flüssigkeitssäule der Reinigungsflüssigkeit als elektrisches Leitungselement vorgenommen werden.

Dazu umfasst das Verteilerelement 52 ein elektrisch isolierend ausgeführtes Außengehäuse 60, das im Ausführungsbeispiel aus einem Kunststoff hergestellt ist. In dem Außengehäuse 60 ist ein ringförmig ausgeführter Medienringkanal 62 angeordnet, der eine Anzahl von entlang des Ringumfangs verteilt angeordneten Medienaustrittsöffnungen 64 aufweist. Die Ringkanalstruktur ist dabei im Ausführungsbeispiel insbesondere aus dem Grund gewählt, dass der im Gehäuse 60 angeordnete Medienringkanal 62 auf den Grundkörper 34 des Befestigungselements 32 aufschiebbar ist, so dass im montierten Zustand des Reinigungssystems 30 eine über den gesamten Umfang des inserierten Pfostenteils 2 hinweg gesehene wirksame Beaufschlagung der äußeren Oberfläche des ersten Dental-Implantat-Teils 2 mit der Reinigungsflüssigkeit erfolgen kann.

Wie der Darstellung in FIG. 3 entnehmbar ist, sind im gezeigten Ausführungsbeispiel die Medienaustrittsöffnungen 64 derart positioniert und angeordnet, dass die austretende Reinigungsflüssigkeit außen an der Außenoberfläche des inserierten Pfostenteils entlangströmt. Zudem ist im dargestellten Beispiel die im montierten Zustand untere Seite des Grundkörpers 34 derart ausgeführt, dass sie näherungsweise plan auf der oberen Stirnseite des inserierten Pfostenteils aufsitzt. In einer alternativen Ausführungsform können diese Komponenten aber auch derart konfiguriert sein, dass der Grundkörper 34 in Umfangsrichtung gesehen zumindest teilweise unter Spaltbildung über der Stirnseite des inserierten Pfostenteils 2 positioniert ist, so dass die Durchströmung eines Mediums ermöglicht ist. Bei einer derartigen Ausführungsform kann der Grundkörper 34 zudem mit Durchtrittskanälen versehen sein, durch die die Reinigungsflüssigkeit ausgehend vom Verteilerelement 52 in den Aufnahmekanal 12, also in den Zwischenraum zwischen der Innenoberfläche des Pfostenteils 2 und dem Grundkörper 34 eintreten, und diesen somit ebenfalls reinigen kann. Durch den zwischen Grundkörper 34 und die Stirnseite des Pfostenteils 2 gebildeten Spalt kann die Reinigungsflüssigkeit anschließend in den Außenbereich des Pfostenteils 2 gelangen und dabei dort ihre Reinigungswirkung in der oben beschriebenen Weise entfalten.

Der Medienringkanal 62 ist medienseitig mit einem Medienkanal 66 verbunden, der seinerseits an das externe Medienreservoir, gegebenenfalls unter Zwischenschaltung von Pumpenanordnungen oder dergleichen, anschließbar ist.

Der Medienringkanal 62 ist somit einerseits auf die Funktion ausgerichtet, mittels der im Umfang verteilt angeordneten Austrittsöffnungen 64 die einströmende Reinigungsflüssigkeit möglichst gleichmäßig um das Pfostenteil oder erste Dental-Implantat-Teil 2 zu verteilen. Im Hinblick auf das Auslegungskonzept des Reinigungssystems 30, dass nämlich die Behandlung des Pfostenteils oder ersten Dental-Implantat-Teils 2 durch eine kombinierte Behandlung durch die Reinigungsflüssigkeit einerseits und einen Stromfluss durch seine Oberfläche andererseits erfolgen soll, ist das Medienkanalsystem für die Reinigungsflüssigkeit, umfassend den Medienkanal 66 und den Medienverteilerring 62, darüber hinaus aber auch noch dafür ausgelegt, die Gegenelektrode zum Pfostenteil oder ersten Dental-Implantat-Teil 2 darzustellen. Im Endbereich der Medienzufuhr, also insbesondere im Bereich der Austrittsöffnungen 64, soll dabei der Stromfluss auf Grund der elektrischen Leitfähigkeit der Reinigungsflüssigkeit an sich ermöglicht werden. Um aber hierfür die elektrische Kontaktierung besonders zuverlässig und auch effizient zu gestalten, kann das Medienkanalsystem zusätzlich in der Art einer Elektrode ausgeführt sein. Dazu kann einerseits in den Medienkanal 66 ein Stromkabel integriert sein, dessen freiliegendes, elektrisch leitfähiges Ende im Bereich des Medienverteilerrings 62 positioniert sein kann. Alternativ oder zusätzlich ist für eine besonders wirksame Unterstützung der Elektrodenfunktion des Mediensystems die Innenoberfläche des Medienkanals 66 und/oder des Medienverteilerrings 62 elektrisch leitfähig, beispielsweise metallisch ausgeführt. Im Hinblick auf eine erwünschte hohe Biokompatibilität, die die Verwendung bei der Patientenbehandlung besonders begünstigt, ist der Medienverteilerring 62 vorzugsweise aus Titan Grade 5 gefertigt.

Weiterhin sind der Medienkanal 66 und insbesondere auch der Medienverteilerring 62 an seiner Innenoberfläche mit einer metallischen Beschichtung 70, im Ausführungsbeispiel aus Aluminium und/oder Magnesium, ausgeführt. Diese Beschichtung 70 stellt einerseits besonders zuverlässig die gewünschten elektrischen Leiteigenschaften für das Medienzuführsystem bereit, so dass dieses auf besonders einfache und zuverlässige Weise als Gegenelektrode verwendet werden kann. Um darüber hinaus aber auch die Auslegung des Verteilerelements 52 als Einwegprodukt besonders zu begünstigen, ist die metallische Beschichtung 70, beispielsweise mittels einer geeigneten Wahl der Beschichtungsdicke, als metallische Opferschicht ausgeführt. Diese Auslegung bedeutet, dass sich beim betrieblichen Gebrauch des Verteilerrings 52, also der Zuführung der Reinigungsflüssigkeit bei gleichzeitiger elektrischer Bestromung, die metallischen Bestandteile der Opferschicht in der zugeführten Reinigungsflüssigkeit elektrochemisch auflösen und diese dabei mit Metallionen anreichern, die den Reinigungs- und Bestromungsprozess des Pfostenteils oder ersten Dental-Implantat-Teils 2 besonders begünstigen.

Sobald diese als Opferschicht ausgeführte metallische Beschichtung 70 in Folge dieser elektrochemischen Auflösung in Folge der Behandlung abgetragen ist, steht nur noch das Basismaterial des Flüssigkeitsverteilerrings 62 bzw. des Medienkanals 66 in Kontakt mit der darin geführten Reinigungsflüssigkeit, und die elektrochemische Aktivität des Systems ist erheblich eingeschränkt. Bei geeigneter Wahl der Betriebsparameter (beispielsweise Stromdichte, Temperatur, Zusammensetzung der Reinigungsflüssigkeit) ist damit nach vollständigem Abtrag der Opferschicht eine weitere Verwendung des Verteilerelements 52 nicht mehr möglich, und damit kann dieses auf besonders günstige Weise für eine lediglich einmalige Verwendung für die Patientenbehandlung ausgelegt sein.

Das Reinigungssystem 30 ist besonders bevorzugt für eine Behandlung des Dental-Implantat-Teils 2 unter Verwendung einer Reinigungsflüssigkeit vorgesehen, wie sie in ihren Bestandteilen und Varianten aus der DE 10 2012 022 593 bekannt ist. Besonders gute und damit vorteilhafte Reinigungsergebnisse lassen sich damit nämlich erreichen, indem eine kombinierte Anwendung einer derartigen Reinigungsflüssigkeit bei zusätzlicher Bestromung des Dental-Implantat-Teils 2 erfolgt. Durch die Ausgestaltung der metallischen Beschichtung als Opferschicht, bei der ein entsprechender loneneintrag in den zugeführten Elektrolyten erfolgt, kann eine solchermaßen ausgelegte Reinigungsflüssigkeit in der Art einer in situ-Erzeugung während der eigentlichen Verfahrensführung generiert werden. In einer besonders bevorzugten und als eigenständig erfinderisch angesehene Verwendung des Reinigungssystems 30 wird dem Verteilerelement 52 dabei ein Elektrolyten, umfassend Zitronensäure, Milchsäure, Äpfelsäure und/oder Essigsäure, zugeführt. Durch diese besonders bevorzugte kombinierte Verwendung des Elektrolyten mit dem Reinigungssystem 30 entsteht durch den Ioneneintrag aus der Opferschicht in den Elektrolyten hinein die eigentlich im inserierten Bereich des Implantats 2 wirksame Behandlungs- und Reinigungsflüssigkeit, die zusätzlich zu den genannten Säureanteilen den lonenanteil aus Magnesium, Aluminium, Mangan, Zink und/oder Kupfer umfasst, und die in ihren Bestandteilen und Varianten aus der
DE 10 2012 022 593 bekannt ist. Gerade eine diese Bestandteile umfassende Reinigungsflüssigkeit hat sich in überraschender Weise als besonders zuverlässig und wirksam bei der Behandlung des inserierten Implantat-Teils erwiesen. Vorzugsweise kann dabei der dem Verteilerelement 52 zugeführte Elektrolyt auch bereits von vornherein Zusatzanteile eines Metallsalzes, umfassend Magnesium-, Aluminium-, Kalium- und/ oder Calciumionen, enthalten.

Grundsätzlich sind im Ausführungsbeispiel die zur Stromführung vorgesehenen Bestandteile des Reinigungssystems 30 aus Titan, bevorzugt aus Reintitan (grade 1 bis 4), als Basismaterial gefertigt, da dieses aufgrund seiner hohen Biokompatibilität besonders zuverlässig für die genannten Zwecke einsetzbar ist. Um dabei auch im Hinblick auf die bevorzugt vorgesehene Ausgestaltung als Einwegprodukt auf einfache Weise eine besonders hohe Bediensicherheit zu gewährleisten, sind diese Komponenten zudem in der Art einer gekapselten Ausführung mit einer wasserlöslichen Außenbeschichtung 72, beispielsweise aus Zucker, versehen, die sich im Kontakt mit der Reinigungsflüssigkeit oder einer Körperflüssigkeit auflöst und das eigentliche Elektrodenmaterial freigibt. Die Außenbeschichtung 72 ist dabei direkt auf dem Basismaterial oder gegebenenfalls auch auf der auf diesem vorgesehenen metallischen Beschichtung 70 in der Art eines Schichtpakets aufgebracht.

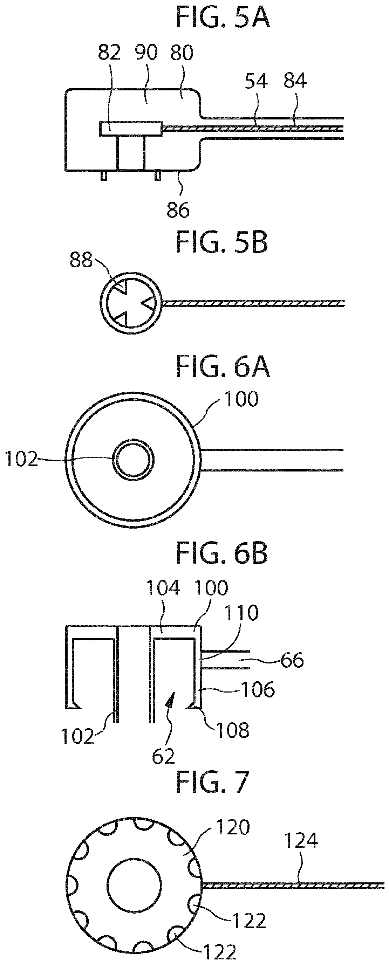

Der innere Aufbau des Kontaktelements 50 wird aus den Darstellungen gemäß FIG. 5 deutlich. FIG. 5A zeigt das Kontaktelement 50 im Längsschnitt. Das Kontaktelement 50 umfasst einen äußeren Gehäusekörper 80, der vorzugsweise aus einem Kunststoff oder einem anderen elektrisch isolierenden Material gebildet ist. In den Gehäusekopf 80 ist ein elektrisch leitfähiger, vorzugsweise metallischer, Kontaktklemmring 82 integriert, der an ein die Stromleitung 54 bildendes, nach außen führendes elektrisches Leiterelement 84 angeschlossen ist. Der Kontaktklemmring 82 ist ringförmig ausgeführt und derart dimensioniert, dass er geeignet auf den Grundkörper 34 des Befestigungselements 32 aufgeschoben werden kann. An der unteren Endfläche 86 des Gehäusekopfs 80 ist eine Anzahl von Steckerelementen 88 angeformt, die in zugeordnete, geeignet ausgeführte Buchsen auf der Oberseite des Verteilerelements 52 einrasten können. Bei der mehrteiligen Ausführung des Reinigungssystems 30 kann somit das Kontaktelement 50 beim Aufstecken auf das Befestigungselement 32 rastend mit dem Verteilerelement 52 verbunden werden.

Wie der Querschnittsdarstellung in FIG. 5B entnehmbar ist, ist an den Kontaktklemmring 82 eine Anzahl von nach innen weisenden Kontaktierungsspitzen 88 angeformt. Beim Aufstecken des Kontaktelements 50 auf das Befestigungselement 32 durchstoßen diese die auf dem Grundkörper 34 angebrachte Beschichtung und stellen somit eine zuverlässige elektrische Verbindung des Kontaktklemmrings 82 zum Grundkörper 34 des Befestigungselements 32 sicher. Des Weiteren sind die Kontaktierungsspitzen 88 im Hinblick auf ihre Materialwahl und Dimensionierung derart ausgeführt, dass sie sich beim Aufstecken auf das Befestigungselement 32 verformen und/oder verbiegen. Damit ist nach dem Gebrauch des Kontaktelements 50 erkennbar, dass dieses bereits verwendet wurde, so dass die Auslegung als Einwegprodukt besonders begünstigt ist.

Um die Verwendung als Einwegprodukt noch weiter zu begünstigen, ist der zentrale obere Bereich 90 des Gehäusekopfs 80 als durchstoßbares Siegel ausgeführt. Dieser Auslegung liegt das Konzept zu Grunde, dass die Montage des Reinigungssystems 30 derart erfolgen soll, dass zunächst das Befestigungselement 32 auf das Pfostenteil oder erste Dental-Implantat-Teil 2 aufgeschraubt wird und anschließend das Verteilerelement 52 und das Kontaktelement 50 rastend auf dieses aufgesteckt werden. Bei der Demontage des Reinigungssystems 30 soll sodann das im oberen zentralen Bereich 90 des Gehäusekopfs 80 befindliche Siegel durch ein geeignetes Werkzeug, beispielsweise einen Schraubendreher, durchstoßen werden, so dass das Werkzeug in eine zugeordnete Nut 92 im Kopfbereich des Grundkörpers 34 des Befestigungselements 32 eingreifen kann. Damit kann mit Hilfe des Werkzeugs das Befestigungselement 32 mitsamt der darauf aufgesteckten Komponentenkontaktelement 50 und Verteilerelement 52 aus dem Gewinde 20 des Pfostenteils herausgeschraubt werden. Durch das dafür notwendige Durchstoßen des Siegels ist sichergestellt, dass das Kontaktelement 50 lediglich für eine einmalige Verwendung geeignet und somit als Einwegprodukt ausgelegt ist.

Um diese Auslegung noch weiter zu begünstigen, ist im Ausführungsbeispiel auch das Verteilerelement 52 mehrkomponentig ausgeführt, wie dies den Darstellungen in FIG. 6 und FIG. 7 entnehmbar ist. Das Verteilerelement 52 umfasst dazu seinerseits eine Gehäusekappe 100 aus elektrisch isolierendem Material, vorzugsweise aus Kunststoff. Wie der Darstellung im Querschnitt in FIG. 6A und im Längsschnitt in FIG. 6B entnehmbar ist, ist die Gehäusekappe 100 ihrerseits ringförmig aufgebaut und umfasst einen inneren Zylindermantel 102, der auf den Grundkörper 34 des Befestigungselements 32 aufschiebbar ist. Am oberen Ende des Zylindermantels 102 ist ein auskragender Deckelring 104 angeformt, der in seinem Außenbereich in einen den Zylindermantel 102 ringförmig umschließenden äußeren Zylindermantel 106 übergeht. Der äußere Zylindermantel 106 läuft in seinem unteren Ende in einen umlaufenden Rastkragen 108 aus, der beim Aufstecken der Gehäusekappe 100 auf das Befestigungselement 32 auf dessen umlaufende Ringwulst 40 einrastet. Im aufgesteckten Zustand bilden somit Zylindermäntel 102, 106 gemeinsam mit dem Deckelring 104 und der Ringwulst 40 den Ringkanal 62 des Verteilerelements 52, der medienseitig über eine Durchtrittsöffnung 110 mit dem am äußeren Zylindermantel 106 angeformten Medienkanal 66 verbunden ist.

In den zur Bildung des Ringkanals 62 vorgesehenen Zwischenraum zwischen innerem Zylindermantel und äußerem Zylindermantel 106 ist ein in FIG. 7 im Querschnitt gezeigter Flüssigkeitsverteiler 120, vorzugsweise aus einem elektrisch leitenden Material, eingelegt, der in seinem Außenbereich eine Anzahl von im eingebauten Zustand mit den Austrittsöffnungen 64 korrespondierenden Einkerbungen 122 zur gezielten Verteilung der Reinigungsflüssigkeit versehen ist. Der Verteilerring 120 ist im Ausführungsbeispiel elektrisch leitend über ein Stromkabel 124 mit der externen Stromquelle verbindbar.

### Bezugszeichenliste

- 1: Dental-Implantatsystem
- 2, 4: Dental-Implantat-Teil
- 6: Außengewinde
- 8: apikales Ende
- 10: Verbindungszapfen
- 12: Aufnahmekanal
- 18: Verbindungsschraube
- 20: Schraubgewinde
- 30: Reinigungssystem
- 32: Befestigungselement
- 34: Grundkörper
- 36: Ende des Grundkörpers 34
- 38: Außengewinde des Grundkörpers 34
- 40: Ringwulst
- 42: elektrisch isolierende Oberflächenbeschichtung
- 50: Kontaktelement
- 52: Verteilerelement
- 54: Stromleitung
- 56: Kontaktkopf
- 60: Außengehäuse des Verteilerelements 52
- 62: Medienringkanal/Medienverteilerring/Ringkanal
- 64: Medienaustrittsöffnung des Medienringkanals 62
- 66: Medienkanal
- 70: metallische Beschichtung
- 72: Außenschicht
- 80: Gehäusekörper
- 82: Kontaktklemmring
- 86: Endfläche
- 88: Steckerelement
- 90: zentraler oberer Bereich des Gehäusekopfs 80
- 92: Nut
- 100: Gehäusekappe des Verteilerelements 52
- 102: Zylindermantel der Gehäusekappe 100
- 104: Deckelring des Zylindermantels 104
- 106: äußerer Zylindermantel
- 108: Rastkragen des äußeren Zylindermantels 106
- 110: Durchtrittsöffnung des Ringkanals 62
- 120: Flüssigkeitsverteiler
- 122: Einkerbungen des Flüssigkeitsverteilers 120
- 124: Stromkabel

## Patentansprüche

1. Reinigungssystem (30) für ein in den Kieferknochen eines Patienten eingebrachtes Dental-Implantat-Teil (2), mit einem Befestigungselement (32), mit einem Medienkanal (66), mit einem über diesen Medienkanal (66) mit einem externen Medienreservoir verbindbaren Verteilerelement (52) für eine Reinigungsflüssigkeit, und mit einem elektrischen Kontaktelement (50), über das eine an eine externe Stromquelle anschließbare Stromleitung (54) elektrisch mit dem Dental-Implantat-Teil (2) verbindbar ist, wobei das Befestigungselement (32) mit seinem Verbindungsende an ein zugeordnetes Verbindungselement im Dental-Implantat-Teil (2) angepasst ist, wobei auf das Befestigungselement (32) das Verteilerelement (52) und zusätzlich zu diesem das elektrische Kontaktelement (50) aufsteckbar sind, **dadurch gekennzeichnet, dass** der Medienkanal (66) und/oder ein mit diesem verbindbarer Medienringkanal (62) an seiner Kanaloberfläche mit einer metallischen Opferschicht (70) versehen ist.

2. Reinigungssystem (30) nach Anspruch 1, dessen Befestigungselement (32) einen elektrisch leitfähigen, vorzugsweise metallischen, Grundkörper (34) aufweist, der mit einer elektrisch isolierenden Oberflächenbeschichtung (42) versehen ist.

3. Reinigungssystem (30) nach Anspruch 1 oder 2, dessen Verteilerelement (52) einen medienseitig mit dem Medienkanal (66) verbindbaren Medienringkanal (62) mit einer Anzahl von Medienaustrittsöffnungen (64) aufweist.

4. Reinigungssystem (30) nach einem der Ansprüche 1 bis 3, dessen Verteilerelement (52) ein elektrisch isolierendes Außengehäuse (60) aufweist.

5. Reinigungssystem (30) nach einem der Ansprüche 1 bis 4, in dessen Medienkanal (66) ein stromleitendes Kabel (124) integriert ist.

6. Reinigungssystem (30) nach einem der Ansprüche 1 bis 5, dessen Verteilerelement (52) und/oder Kontaktelement (50) als Einwegprodukt ausgebildet ist.

7. Reinigungssystem (30) nach einem der Ansprüche 1 bis 6, dessen Opferschicht (70) Magnesium und/oder Aluminium umfasst.

8. Reinigungssystem (30) nach einem der Ansprüche 1 bis 7, bei dem das Außengehäuse (60) des Verteilerelements (52) einen auf eine korrespondierende umlaufende Wulst (40) des Befestigungselements (32) aufsteckbaren Rastkragen (108) aufweist.

9. Reinigungssystem (30) nach einem der Ansprüche 1 bis 8, dessen elektrisches Kontaktelement (50) einen elektrisch leitend mit der Stromleitung verbundenen, mit einer Anzahl von Kontaktierungsspitzen versehenen Kontaktklemmring (82) aufweist.

10. Reinigungssystem (30) nach einem der Ansprüche 1 bis 9, dessen Kontaktelement (50), Verteilerelement (52) und/oder Befestigungselement (32) ganz oder teilweise mit einer wasserlöslichen Außenbeschichtung (72), vorzugsweise aus Zucker, versehen sind.

## Claims

1. A cleaning system (30) for a dental implant part (2) which has been introduced into the jawbone of a patient, having a securing element (32), having a media channel (66) with a distribution element (52) for a cleaning fluid which can be connected to an external media reservoir via said media channel (66) and with an electrical contact element (50) via which a power supply line (54) which can be connected to an external power source can be electrically connected to the dental implant part (2), wherein the connection end of the securing element (32) is adapted to an associated connection element in the dental implant part (2), wherein the distribution element (52) and in addition thereto the electrical contact element (50) can be attached to the securing element (32), **characterized in that** the media channel (66) and/or an annular media channel (62) which can be connected thereto is provided with a metallic sacrificial layer (70) on its channel surface.

2. The cleaning system (30) as claimed in claim 1, in which the securing element (32) has an electrically conductive, preferably metallic base body (34) which is provided with an electrically insulating surface coating (42).

3. The cleaning system (30) as claimed in claim 1 or claim 2, in which the distribution element (52) has an annular media channel (62) having a plurality of media outlet openings (64) which can be connected to the media channel (66) on the media side.

4. The cleaning system (30) as claimed in one of claims 1 to 3, in which the distribution element (52) has an electrically insulating external housing (60).

5. The cleaning system (30) as claimed in one of claims 1 to 4, in which a power cable (124) is integrated into its media channel (66).

6. The cleaning system (30) as claimed in one of claims 1 to 5, in which the distribution element (52) and/or contact element (50) is configured as a disposable product.

7. The cleaning system (30) as claimed in one of claims 1 to 6, in which the metallic sacrificial layer (70) comprises magnesium and/or aluminium.

8. The cleaning system (30) as claimed in one of claims 1 to 7, in which the external housing (60) of the distribution element (52) has a detent collar (108) which can be attached to a corresponding peripheral bead (40) of the securing element (32).

9. The cleaning system (30) as claimed in one of claims 1 to 8, in which the electrical contact element (50) has a contact clamping ring (82) which is connected in an electrically conductive manner to the power supply line and is provided with a plurality of contact points.

10. The cleaning system (30) as claimed in one of claims 1 to 9, in which all or a portion of the contact element (50), distribution element (52) and/or securing element (32) is provided with a water-soluble external coating (72), preferably produced from sugar.

## Revendications

1. Système de nettoyage (30) d'une pièce d'implant dentaire (2) introduite dans la mâchoire d'un patient, pourvu d'un élément de fixation (32), d'un canal à fluide (66), d'un élément distributeur (52) d'un liquide nettoyant susceptible d'être relié par l'intermédiaire dudit canal à fluide (66) avec un réservoir à fluide externe, d'un élément de contact (50) électrique, par l'intermédiaire duquel un conduit électrique (54) susceptible d'être raccordé sur une source électrique externe est susceptible d'être relié avec la pièce d'implant dentaire (2), l'élément de fixation (32) étant adapté par son extrémité de liaison à un élément de liaison associé dans la pièce d'implant dentaire (2), sur l'élément de fixation (32) étant enfichable l'élément distributeur (52) et en sus de celui-ci, l'élément de contact (50) électrique, **caractérisé en ce que** le canal à fluide (66) et/ou un canal annulaire à fluide (62) susceptible d'être relié avec celui-ci est muni sur sa surface de canal d'une couche sacrificielle (70) métallique.

2. Système de nettoyage (30) selon la revendication 1, dont l'élément de fixation (32) comporte un corps de base (34) conducteur d'électricité, de préférence métallique, qui est muni d'un revêtement superficiel (42) isolant électrique.

3. Système de nettoyage (30) selon la revendication 1 ou 2, dont l'élément distributeur (52) comporte un canal annulaire à fluide (62) susceptible d'être relié du côté fluide avec le canal à fluide (66), pourvu d'un nombre d'orifices de sortie de fluide (64).

4. Système de nettoyage (30) selon l'une quelconque des revendications 1 à 3, dont l'élément distributeur (52) comporte un boîtier extérieur (60) isolant électrique.

5. Système de nettoyage (30) selon l'une quelconque des revendications 1 à 4, dans le canal à fluide (66) duquel est intégré un câble (124) conducteur d'électricité.

6. Système de nettoyage (30) selon l'une quelconque des revendications 1 à 5, dont l'élément distributeur (52) et/ou l'élément de contact (50) est conçu sous la forme d'un produit jetable.

7. Système de nettoyage (30) selon l'une quelconque des revendications 1 à 6, dont la couche sacrificielle (70) comprend du magnésium et /ou de l'aluminium.

8. Système de nettoyage (30) selon l'une quelconque des revendications 1 à 7, sur lequel le boîtier extérieur (60) de l'élément distributeur (52) comporte une collerette enclenchable (108) susceptible d'être enfichée sur un bourrelet (40) périphérique correspondant de l'élément de fixation (32).

9. Système de nettoyage (30) selon l'une quelconque des revendications 1 à 8, dont l'élément de contact (50) électrique comporte une bague de serrage de contact (82) reliée de manière conductrice d'électricité avec le conduit électrique, munie d'un nombre de pointes de contact

10. Système de nettoyage (30) selon l'une quelconque des revendications 1 à 9, dont l'élément de contact (50), l'élément distributeur (52) et/ou l'élément de fixation (32) sont munis totalement ou en partie d'un revêtement extérieur (72) hydrosoluble, de préférence en sucre.
